# EUROPEAN PATENT APPLICATION

(11) **EP 2 653 555 A1**
(43) Date of publication of application: **23.10.2013**
(21) Application number: 12164807.5
(22) Date of filing: 19.04.2012
(51) Int. Cl.: C12Q 1/68

(54) **P38 MAPK gamma and delta for use as biomarkers of NAFLD**

(71) Applicant: Fundacion Centro Nacional De Investigaciones Cardiovasculares Carlos III (CINC), 28029 Madrid (ES); Consejo Superior de Investigaciones Cientificas (CSIC), 28006 Madrid (ES); Universidad de Salamanca, 37008 Salamanca (ES)
(72) Inventor: Sabio Buzo, Guadalupe, 28029 Madrid (ES); González Terán, Bárbara, 28029 Madrid (ES); Bernardo Vasco, Edgar, 28029 Madrid (ES); Matesanz Parellada, Nuria, 28029 Madrid (ES); Verdugo Becerra, Mª de los Ángeles, 28049 Madrid (ES); Marcos Martín, Miguel, 37008 Salamanca (ES); Hernández Cosido, Lourdes, 37008 Salamanca (ES); Ortega Martín-Corral, Luis E., 37008 Salamanca (ES)
(74) Representative: Illescas, Manuel

(57) **Abstract**

**p38 MAPK gamma and delta for use as biomarkers of NAFLD.** The present invention provides an *in vitro* method for prognosis and diagnosis NAFLD, preferably for hepatic steatosis. The method of the invention is based on blood samples and consequently it may be considered as a non-invasive test for diagnosis of hepatic steatosis. Blood levels of p38 mapk gamma and delta are therefore used as biomarkers of hepatic steatosis in human.

SEQUENCE LISTING

<110> Fundación Centro Nacional de Investigaciones Cardiovasculares
      Carlos II (CNIC)
<110> Consejo Superior de Investigaciones Científicas (CSIC)
<110> Universidad de Salamanca

<120> P38MAPK GAMMA AND DELTA FOR USE AS BIOMARKERS OF NAFLD

<130> EP-05368

<160> 26

<170> BiSSAP 1.0

<210> 1
<211> 23
<212> DNA
<213> artificial sequences

<220>
<221> source
<222> 1..23
<223> /mol_type="DNA"
      /note="artificial sequences"
      /organism="artificial sequences"

<400> 1
tagtccttcc taccccaatt tcc                                              23


<210> 2
<211> 21
<212> DNA
<213> artificial sequences

<220>
<221> source
<222> 1..21
<223> /mol_type="DNA"
      /note="artificial sequences"
      /organism="artificial sequences"

<400> 2
ttggtcctta gccactcctt c                                                21


<210> 3
<211> 23
<212> DNA
<213> artificial sequences

<220>
<221> source
<222> 1..23
<223> /mol_type="DNA"
      /note="artificial sequences"
      /organism="artificial sequences"

<400> 3
ccctcacact cagatcatct tct                                              23


<210> 4
<211> 19

<212> DNA
<213> artificial sequences

<220>
<221> source
<222> 1..19
<223> /mol_type="DNA"
      /note="artificial sequences"
      /organism="artificial sequences"

<400> 4
gctacgacgt gggctacag                                                    19


<210> 5
<211> 21
<212> DNA
<213> artificial sequences

<220>
<221> source
<222> 1..21
<223> /mol_type="DNA"
      /note="artificial sequences"
      /organism="artificial sequences"

<400> 5
gctcttactg actggcatga g                                                 21


<210> 6
<211> 20
<212> DNA
<213> artificial sequences

<220>
<221> source
<222> 1..20
<223> /mol_type="DNA"
      /note="artificial sequences"
      /organism="artificial sequences"

<400> 6
cgcagctcta ggagcatgtg                                                   20


<210> 7
<211> 21
<212> DNA
<213> artificial sequences

<220>
<221> source
<222> 1..21
<223> /mol_type="DNA"
      /note="artificial sequences"
      /organism="artificial sequences"

<400> 7
gcaactcgga cctggtcata a                                                 21


<210> 8
<211> 19
<212> DNA

<213> artificial sequences

<220>
<221> source
<222> 1..19
<223> /mol_type="DNA"
      /note="artificial sequences"
      /organism="artificial sequences"

<400> 8
cggcccgtga tgagaaact                                                    19


<210> 9
<211> 20
<212> DNA
<213> artificial sequences

<220>
<221> source
<222> 1..20
<223> /mol_type="DNA"
      /note="artificial sequences"
      /organism="artificial sequences"

<400> 9
gagcggagag tactggatcg                                                   20


<210> 10
<211> 20
<212> DNA
<213> artificial sequences

<220>
<221> source
<222> 1..20
<223> /mol_type="DNA"
      /note="artificial sequences"
      /organism="artificial sequences"

<400> 10
gcttcttttc cttggggttc                                                   20


<210> 11
<211> 21
<212> DNA
<213> artificial sequences

<220>
<221> source
<222> 1..21
<223> /mol_type="DNA"
      /note="artificial sequences"
      /organism="artificial sequences"

<400> 11
aggtcggtgt gaacggattt g                                                 21


<210> 12
<211> 19
<212> DNA
<213> artificial sequences

<220>
<221> source
<222> 1..19
<223> /mol_type="DNA"
      /note="artificial sequences"
      /organism="artificial sequences"

<400> 12
ggggtcgttg atggcaaca                                                    19


<210> 13
<211> 20
<212> DNA
<213> artificial sequences

<220>
<221> source
<222> 1..20
<223> /mol_type="DNA"
      /note="artificial sequences"
      /organism="artificial sequences"

<400> 13
agagccccaa gaaggatttc                                                   20


<210> 14
<211> 20
<212> DNA
<213> artificial sequences

<220>
<221> source
<222> 1..20
<223> /mol_type="DNA"
      /note="artificial sequences"
      /organism="artificial sequences"

<400> 14
agcatcttgt cgagcaggtc                                                   20


<210> 15
<211> 18
<212> DNA
<213> artificial sequences

<220>
<221> source
<222> 1..18
<223> /mol_type="DNA"
      /note="artificial sequences"
      /organism="artificial sequences"

<400> 15
atgcgctaca cgcagaca                                                     18


<210> 16
<211> 20
<212> DNA
<213> artificial sequences

<220>
<221> source
<222> 1..20
<223> /mol_type="DNA"
      /note="artificial sequences"
      /organism="artificial sequences"

<400> 16
tggtcattgc ctttgaacag                                                   20


<210> 17
<211> 21
<212> DNA
<213> artificial sequences

<220>
<221> source
<222> 1..21
<223> /mol_type="DNA"
      /note="artificial sequences"
      /organism="artificial sequences"

<400> 17
aggtcggtgt gaacggattt g                                                 21


<210> 18
<211> 19
<212> DNA
<213> artificial sequences

<220>
<221> source
<222> 1..19
<223> /mol_type="DNA"
      /note="artificial sequences"
      /organism="artificial sequences"

<400> 18
ggggtcgttg atggcaaca                                                    19


<210> 19
<211> 21
<212> DNA
<213> artificial sequences

<220>
<221> source
<222> 1..21
<223> /mol_type="DNA"
      /note="artificial sequences"
      /organism="artificial sequences"

<400> 19
ctgaacaaca tcgtcaagtg c                                                 21


<210> 20
<211> 21
<212> DNA
<213> artificial sequences

<220>

<221> source
<222> 1..21
<223> /mol_type="DNA"
      /note="artificial sequences"
      /organism="artificial sequences"

<400> 20
aaccaggaat tgaacgtgct c                                                21


<210> 21
<211> 21
<212> DNA
<213> artificial sequences

<220>
<221> source
<222> 1..21
<223> /mol_type="DNA"
      /note="artificial sequences"
      /organism="artificial sequences"

<400> 21
catgagaagc taggcgagga c                                                21


<210> 22
<211> 21
<212> DNA
<213> artificial sequences

<220>
<221> source
<222> 1..21
<223> /mol_type="DNA"
      /note="artificial sequences"
      /organism="artificial sequences"

<400> 22
gcatctggta cacgaggaac t                                                21


<210> 23
<211> 21
<212> DNA
<213> artificial sequences

<220>
<221> source
<222> 1..21
<223> /mol_type="DNA"
      /note="artificial sequences"
      /organism="artificial sequences"

<400> 23
aaaagggctt ctacaagcag g                                                21


<210> 24
<211> 19
<212> DNA
<213> artificial sequences

<220>
<221> source

<222> 1..19
<223> /mol_type="DNA"
      /note="artificial sequences"
      /organism="artificial sequences"

<400> 24
tcggggacac gtaggtctt                                                    19

<210> 25
<211> 19
<212> DNA
<213> artificial sequences

<220>
<221> source
<222> 1..19
<223> /mol_type="DNA"
      /note="artificial sequences"
      /organism="artificial sequences"

<400> 25
cgatgggcgg cggaaaata                                                    19

<210> 26
<211> 21
<212> DNA
<213> artificial sequences

<220>
<221> source
<222> 1..21
<223> /mol_type="DNA"
      /note="artificial sequences"
      /organism="artificial sequences"

<400> 26
ccacaccctt aatggcagtg a                                                 21

## Description

### FIELD OF THE INVENTION

The present invention may be included in the field of the medicine, in general, and, more particularly, in that area of the medicine which is focused on the study of nonalcoholic fatty liver disease (NAFLD) and, more particularly, of hepatic steatosis, also known as fatty liver, characterized by the abnormal fat accumulation that occurs in the liver cells.

### STATE OF THE ART

Obesity is a major health problem associated with many other diseases like type two diabetes T2DM, metabolic syndrome and NAFLD. In fact, NAFLD is one of the most prevalent manifestations of obesity [1] and this chronic liver disease has become a worldwide health problem.

NAFLD refers to a spectrum of liver diseases ranging from simple steatosis or fatty liver, to nonalcoholic steatohepatitis (NASH), and cirrhosis (with advanced scarring of the liver), all these stages have in common an accumulation of fat within hepatocytes. Most patients with NAFLD also have obesity (60-100%), T2DM (28-55%) and hyperlipidemia (27-92%) [2]. While NAFLD can be quite unobtrusive, NASH is a serious condition, with nearly a quarter of affected patients developing cirrhosis, which, in turn, increases the risk of subsequent progression to hepatocellular carcinoma (HCC). HCC is the fifth most common tumour worldwide with increasing incidence and third leading cause of cancer-related death. Among patients with NAFLD or NASH, HCC represents the main cause of death [3].

It has also been estimated that around 20-30% of the general population have NAFLD [4,5], a asymptomatic disease that can progress from an initial trygliceride accumulation to NASH which, in a long-term, may results in fibrosis, cirrhosis and hepatocelullar carcinoma (HCC) [6, 7, 8]. For that reason, an early and accurate diagnosis is fundamental to know the degree of liver damage, and avoid the disease progression.

Liver biopsy is nowadays considered the gold standard for NAFLD detection, since the diagnostic yield of available non-invasive tests is not enough accurate [9, 10]. Despite of providing important information about liver damage and staging, liver biopsy is an invasive procedure, uncomfortable and with risks for the patient and therefore it cannot be used as a screening or routine method, or for obtaining several samples periodically to assess disease evolution.

Therefore, a reliable and non-invasive test for diagnosis of liver steatosis would lead to a clear improvement in early diagnosis, ability of monitoring of the disease, as well as quality of life.

The present invention solves the above-mentioned problem by providing solid biomarkers (p38MAPK) for NAFLD, preferably for hepatic steatosis. p38MAPK (mitogen-activated protein kinase) families are strongly activated *in vivo* by environmental stress factors and inflammatory cytokines. Obesity and liver steatosis act like stress signals inducing multiple responses in tissues. Four p38MAPK isotypes have been described to date: alpha (α), beta (β), gamma (γ) and delta (δ). The canonical activation of p38MAPK occurs via dual phosphorylation in the activation loop by MKK3 and MKK6 [12].

Consequently the present invention provides an accurate *in vitro* method for prognosis and diagnosis of NAFLD, preferably of hepatic steatosis, offering the opportunity of carrying out an improved treatment at earlier stages of the disease, thus rising the likelihood of definitely treating the disease. For that reason, an early and accurate diagnosis is fundamental to know the degree of liver damage, and avoid the disease progression. On the other hand, the method of the invention is based on blood samples and consequently it may be considered as a non-invasive test which leads to a clear improvement in early diagnosis, ability of monitoring of the disease, as well as quality of life. Blood levels of these kinases (p38MAPK gamma and p38MAPK delta) can be therefore used as potential biomarkers of hepatic steatosis in human, without the inconvenient and risks of liver biopsy and therefore improving early diagnosis and more accurate prognosis of NAFLD and its associated complications or diseases.

### DESCRIPTION OF THE INVENTION

As explained above, the present invention provides an accurate and non-invasive *in vitro* method for prognosis and diagnosis of NAFLD, preferably of hepatic steatosis, offering the opportunity of carrying out an improved treatment at earlier stages of the disease, thus raising the likelihood of definitely treating the disease. The method of the invention is based on blood samples and consequently it may be considered as a non-invasive test for diagnosis of hepatic steatosis which leads to a clear improvement in early diagnosis, ability of monitoring of the disease, as well as quality of life. Blood levels of these kinases could be therefore used as potential biomarkers of hepatic steatosis in human, without the inconvenient and risks of liver biopsy and therefore improving early diagnosis and more accurate prognosis of NAFLD and its complications.

After a large study with both mice and human blood samples (see examples), a statistically significant relation between liver stetatosis induced by obesity with a decrease in expression levels of kinases p38MAPK *δ* and p38MAPK γ was observed in the present invention (p<0.001 or p<0.01 respectably). Therefore, analysis of these kinases can be used as biomarkers of NAFLD, preferably of liver steatosis, in human, without the drawbacks associated to the use of liver biopsy and therefore improving early diagnosis and more accurate prognosis.

Consequently, the first embodiment of the present invention refers to p38MAPK gamma and/or delta for use as biomarkers of NAFLD, preferably of hepatic steatosis.

The second embodiment of the present invention refers to an *in vitro* method for the diagnosis of NAFLD, preferably of hepatic steatosis, that comprises measuring the expression level of p38MAPK gamma and/or delta in a blood sample taken from the subject under examination. As explained above, hepatic steatosis is associated with a significant decrease in expression levels of p38MAPK gamma and p38MAPK delta. In a preferred embodiment, the method of the invention is characterized in that the expression level of p38MAPK gamma is measured using the primers of SEQ ID NO: 21 and 22 and the expression level of p38MAPK delta is measured using the primers of SEQ ID NO: 23 and 24.

The third embodiment of the present invention refers to a nucleotide sequence characterized by the SEQ ID NO: 21, 22, 23 or 24. These sequences are used in the present invention as primers for measuring the expression level of p38 gamma and/or p38 delta in the blood samples taken from the patient/subject. Consequently these sequences may be used as primers to make a kit for diagnosis of NAFLD, preferably for diagnosis of hepatic steatosis. Thus, in a preferred embodiment the present invention refers to a nucleotide sequence characterized by the SEQ ID NO: 21, 22, 23 or 24 or any combination thereof for use as primers for hybridizing under stringent conditions with a nucleic acid encoding for p38MAPK gamma and/or delta sequence or any partial complementary or mutated sequence thereof.

Thus, the fourth embodiment of the present invention refers to a kit for the diagnosis of NAFLD, preferably of hepatic steatosis, characterized by comprising the primers of SEQ ID NO: 21 and 22 for measuring the expression level of p38MAPK gamma and/or the primers of SEQ ID NO: 23 and 24 for measuring the expression level of p38MAPK delta.

For the purpose of the present invention p<0.05 refers to the significance level and p values below to 0.05 make the biomarkers of the invention statistically significant.

### DESCRIPTION OF THE FIGURES

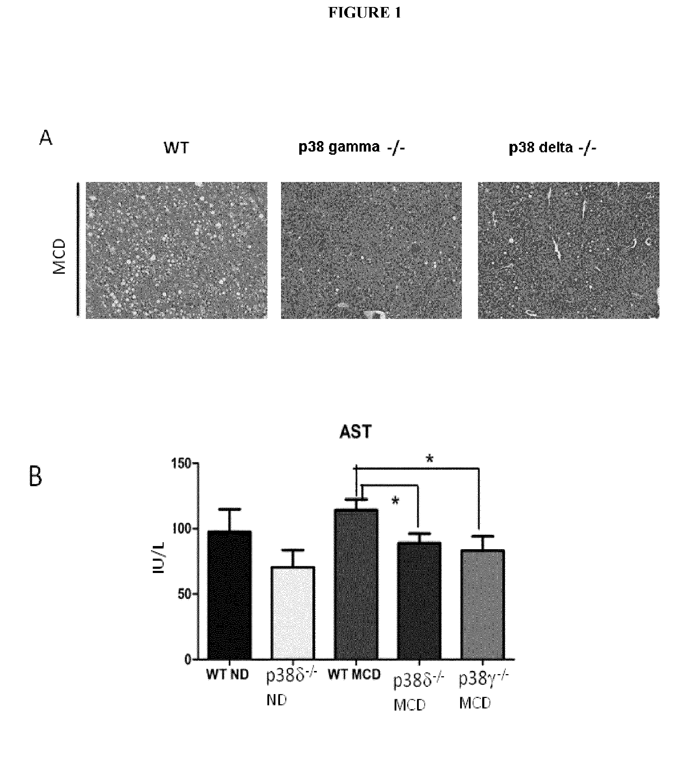

**Figure 1****.** Effect of MCD (methionine choline deficiente) diet in mice lacking p38γ (p38gamma -/-) or p38δ (p38delta -/-). WT (wild type) animal or lacking in p38γ or p38δ were fed for 4 weeks with MCD or control diet (ND). **A)** Histological section of liver stained with hematoxilin and eosin. **B)** AST (Aspartate Aminotransferase) (well known marker of hepatitis necrosis) levels in plasma as indicator of liver damage. Both knock-out mice showed a partial protection against liver steatosis compared with WT animals, since a decrease in the accumulation of lipid droplets in liver is observed. *p < 0.05, **p < 0.01, and ***p < 0.001;by *t*-test.
**Figure 2****.** Effect of MCD diet or ND diet in mice lacking p38 gamma and delta p38δ/γ^{-/-}. WT animals or p38δ/γ^{-/-} were fed for 4 weeks with MCD diet. A) Histological sections of liver stained with hematoxylin and eosin. B) AST and ALT (alanine aminotransferase) in the plasma as indicators of liver damage. Double knock-out mice p38δ/γ^{-/-} are protected against liver steatosis. *p < 0.05,**p < 0.01, and ***p< 0.001; by t-test.
**Figure 3****.**p38δ/γ^{-/-} animals are protected animals against oxidative stress. Triglycerides stored in the liver and lipid peroxidation (Tbars) in livers of WT mice and p38δ/γ^{-/-} fed or not with MCD diet. *p < 0.05,**p < 0.01, and ***p < 0.001;by *t*-test.
**Figure** 4. Lack of p38δ and p38 γ protects animals from inflammation induced by a methionine and choline deficient diet. Liver mRNA levels of TNF, IL-6 and IL-10 in WT mice and p38δ/γ^{-/-} fed with MCD or control diet (ND). *p < 0.05,**p < 0.01, and ***p < 0.001;by t-test.
**Figure 5****.** p38δ/γ^{-/-} animals are protected against MCD-induced fibrosis. Measurement of liver mRNA levels ofTIMPI and collagen from WT and p38δ/γ^{-/-}mice fed MCD or control diet (ND). *p < 0.05,**p < 0.01, and ***p < 0.001;by t-test.
**Figure 6****.** BIRB796 protects animals of MCD-induced steatosis. WT animals were injected with DMSO or BIRB796 daily for 3 weeks and fed MCD diet. Histological sections of liver stained with hematoxylin and eosin reveal that BIRB796 protects against development of steatosis.
**Figure 7****.** p38δ/γ^{-/-} does not develop hepatic steatosis and insulin resistance. WT or p38δ/γ^{-/-} mice fed with normal diet (ND) or high fat diet (HFD) were fasted for 16hrs and then treated with insulin (1.5U/kg). Liver extracts were analyzed for phosphorylation of AKT 308 and 473. Histological sections of liver stained with hematoxylin and eosin revealed that lack of p38δ and p38γ protects animals against development of steatosis.
**Figure 8****.** mRNA levels of p38δ and p38γ decrease during obesity. Relative expression levels respect to GAPDH of p38δ and p38γ in blood from animals fed 20 weeks with high fat diet (HFD) or control diet (ND). *p < 0.05,**p < 0.01, and ***p < 0.001;by t-test.
**Figure 9****.** Levels of hepatic steatosis in control (A) and obese patients (B). Histological sections stained with hematoxylin and eosin revealed different grades of steatosis in obese patients. Histological grading of inflammation and fibrosis was estimated from histological samples. * < 0.05,**p < 0.01, and ***p < 0.001;by *t-*test.
**Figure 10****.** mRNA levels of blood from obese patients and controls. The expression levels of p38gamma and p38delta in whole blood are significantly decreased in obese patients when compared to healthy controls. Expression level of s18 was used as control. *p < 0.05,**p < 0.01, and ***p < 0.001;by *t*-test.

### EXAMPLES

### Example 1. Partial protection of liver damage in p38δ^{-/-} and p38γ^{-/-} mice.

Mice p38δ^{-/-} and p38γ^{-/-} were obtained from Dr. Ana Cuenda laboratory, and housed at the facilities of the Centro Nacional de Biotecnologia (CNB). 10 males (8-10 weeks old) per group were fed with a methionine and choline deficient diet (MCD, Harlan ref TD.90262) or chow diet for 4 weeks. Mice were genotyped by PCR analysis using genomic DNA. Animal studies were approved by the local ethics committee, and all procedures followed EU Directive 86/609/EEC and Recommendation 2007/526/EC regarding the protection of animals used for experimental and other scientific purposes, enacted under Spanish law 1201/2005.

Tissue fixed in 10% formalin for 24 h, dehydrated, and embedded in paraffin was cut in sections (8 µm) and stained using hematoxylin and eosin or Masson trichromic.

Different histological parameters were analyzed on each liver sample.
- Steatosis: Degree, type and localization.
   ● Degree: Presence of lipid accumulation in vesicles, Mild (<10%), Moderate (10-25%), Severe (26-50%) and Massive (>50%).
   ● Type: Microvesicular, Macrovesicular, and Mixed.
   ● Localization: Periportal, Centrilobular, Diffuse.
- Inflammation: Type and degree.
- Fibrosis: Absent, Perisinusoidal.
- Typical hepatocellular lesions: Presence or absence.

The serum concentration of alanine transaminases (ALT) and aspartate aminotransferase (AST) activity was measured using Biosystems reagents (ref 11568 and 11561) and read in a microplate spectrophotometer at 340 nm (Benchmark Plus BioRad) after 10 min.

Statistical significance to compare treatment groups was calculated by Student t-test using Prism software (GraphPad). Data were presented as mean±SEM.

Since obesity is a disease associated to a chronic inflammatory state, characterized by increased inflammatory markers such as C reactive protein, IL-6, IL-8, IL-10, PAI-1, TNF-α, MCP-1 and hepatocyte growth factor, and, moreover, it is well established that the p38MAPK family plays a key role during the inflammatory process, thus, it was decided to study whether p38γ and p38δ could have a role in the development of liver stetaosis. With that aim, p38δ^{-/-}, p38γ^{-/-} and WT mice were fed with a diet deficient in methionine and choline (MCD) for 4 weeks, a diet described to induce liver stetaosis that resembles human NAFLD [13]. Both knock-out mice showed a partial protection against liver steatosis compared with WT animals, since a decrease in the accumulation of lipid droplets in liver was observed **(****Figure 1****).** Lower AST levels, a well-established marker of hepatic necrosis, were also detected in plasma of p38δ^{-/-}, p38γ^{-/-} mice. *p < 0.05,**p < 0.01, and ***p < 0.001;by *t*-test.

### Example 2. p38δ/γ^{-/-} mice are protected against liver steatosis.

Mice p38γ/*δ*^{-/-} were obtained from Dr. Ana Cuenda laboratory, and housed at the facilities of the Centro Nacional de Biotecnologia (CNB). 10 males (8-10 weeks old) per group were fed with a high fat diet (HFD, Harlan ref TD110418) or chow diet for 20 weeks. Mice were genotyped by PCR analysis using genomic DNA. For the inhibition of p38 MAPK isoforms C57BL6J male mice housed at the Centro de Investigaciones Cardiovasculares (CNIC) were used. Animal studies were approved by the local ethics committee, and all procedures followed EU Directive 86/609/EEC and Recommendation 2007/526/EC regarding the protection of animals used for experimental and other scientific purposes, enacted under Spanish law 1201/2005. Tissue fixed in 10% formalin for 24 h, dehydrated, and embedded in paraffin was cut in sections (8 µm) and stained using hematoxylin and eosin or Masson trichromic.

Different histological parameters were analyzed on each liver sample.
- Steatosis: Degree, type and localization.
   ● Degree: Presence of lipid accumulation in vesicles, Mild (<10%), Moderate (10-25%), Severe (26-50%) and Massive (>50%).
   ● Type: Microvesicular, Macrovesicular, and Mixed.
   ● Localization: Periportal, Centrilobular, Diffuse.
- Inflammation: Type and degree.
- Fibrosis: Absent, Perisinusoidal.
- Typical hepatocellular lesions: Presence or absence.

The serum concentration of alanine transaminases (ALT) and aspartate aminotransferase (AST) activity was measured using Biosystems reagents (ref 11568 and 11561) and read in a microplate spectrophotometer at 340 nm (Benchmark Plus BioRad) after 10 min. 25 mg of liver tissue was homogenized in a chloroform and methanol solution (8:1 ratio) and incubated for 4 hours at 22°C. Sulfuric acid 1M was added to the extracts, and samples were centrifuged at 1000 r.p.m. for 10 min. Lipids in the organic phase were transferred to a new tube, quantified using Serum Triglyceride determination Kit (Sigma ref TR0100), and detected after 15 min incubation at 540 nm.

Lipid peroxidation in tissue samples was determined by the thiobarbituric acid reactive substances (TBARS) assay. 25 mg of liver tissue was homogenized in lysis buffer with protease inhibitors and then sonicated. This homogenate was centrifuged and supernatant used for analysis following the manufacture instructions of TBARS assay kit (Cayman ref 10009055).

The expression of mRNA was examined by quantitative PCR analysis using a 7900 Fast Real-Time PCR machine. Primers were produced by Sigma Aldrich **(Table 1).** The relative mRNA expression was normalized by measurement of the amount of *Gapdh* mRNA.

**Table 1**

| | Forward | Reverse |
|---|---|---|
| *mmu-Il6* | SEQ ID NO: 1 | SEQ ID NO: 2 |
| *mmu-Tnf-α* | SEQ ID NO: 3 | SEQ ID NO: 4 |
| *mmu-Il10* | SEQ ID NO: 5 | SEQ ID NO: 6 |
| *mmu-Timp1* | SEQ ID NO: 7 | SEQ ID NO: 8 |
| *mmu-Collagen* | SEQ ID NO: 9 | SEQ ID NO: 10 |
| *mmu-Gapdh* | SEQ ID NO: 11 | SEQ ID NO: 12 |

Statistical significance to compare treatment groups was calculated by Student t-test using Prism software (GraphPad). Data were presented as mean±SEM.

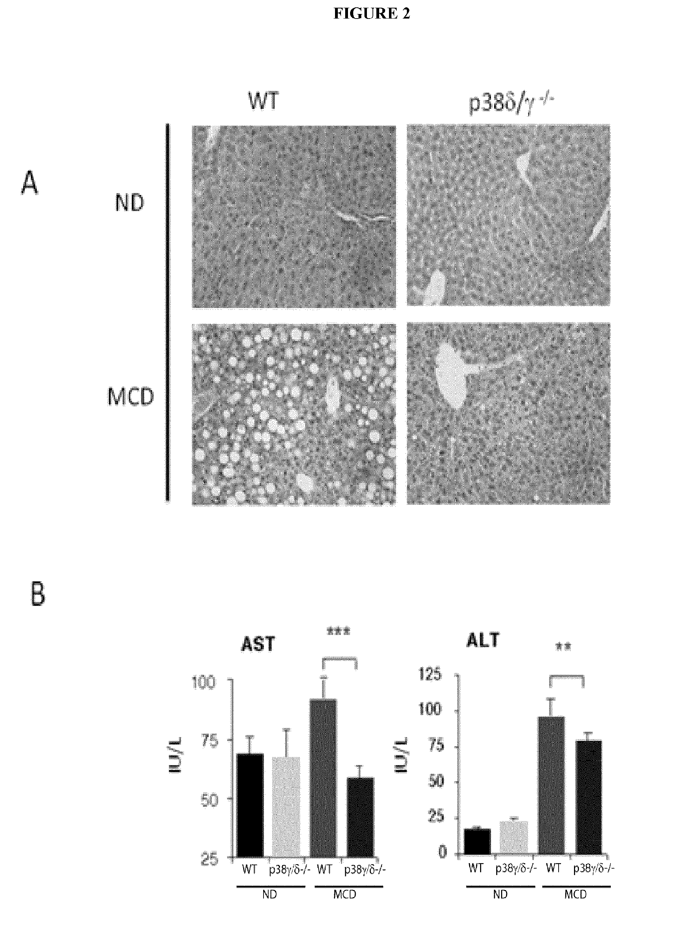

This example showed that p38γ/δ^{Lyz-KO} mice have a greater protection against liver failure than either of the single conditional knock-out, indicating a degree of functional redundancy between p38γ and δ. Then, it was decided to study the effect of lacking the two p38 isoforms (p38γ and δ) in liver steatosis. Double knock-out mice (p38δ/γ-/-) were fed with MCD for four weeks. MCD diet induces a change in liver histology architecture, with an increase in lipids accumulation and leukocyte liver infiltration in WT mice. However, these effects were not present in p38δ/γ^{-/-} mice **(****Figure 2A****)** which correlated with reduced levels of serum transaminases, which are biomarkers of liver necrosis. *p < 0.05,**p < 0.01, and ***p < 0.001;by t-test.

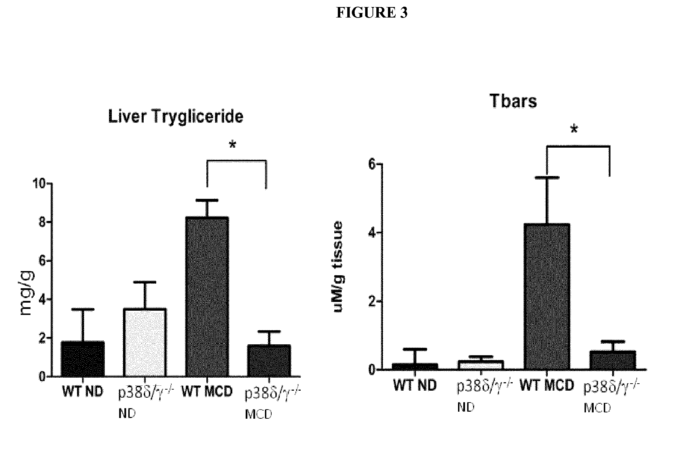

Moreover reduced levels of triglycerides in the liver from p38δ/γ-/- mice correlated with lower lipid peroxidation after MCD diet **(****Figure 3****),** indicating a protection against the oxidative stress that accomplishes the liver steatosis. *p < < 0.05,**p < 0.01, and ***p < 0.001;by t-test

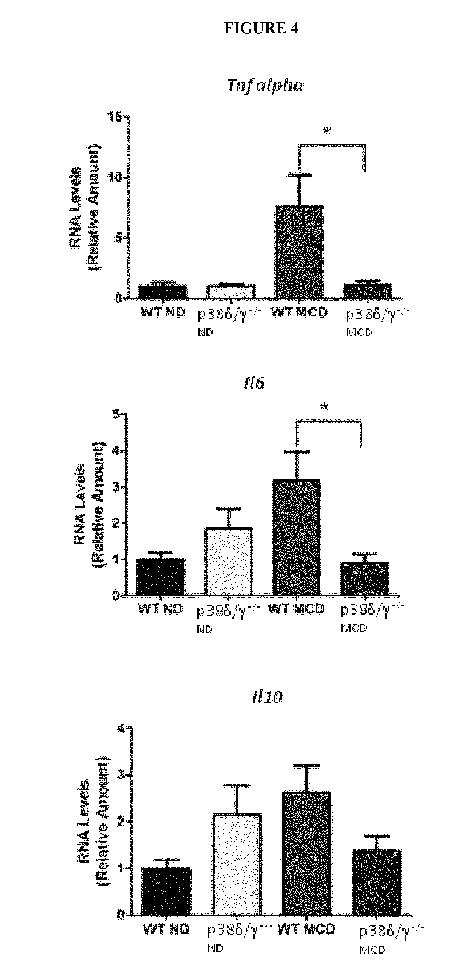

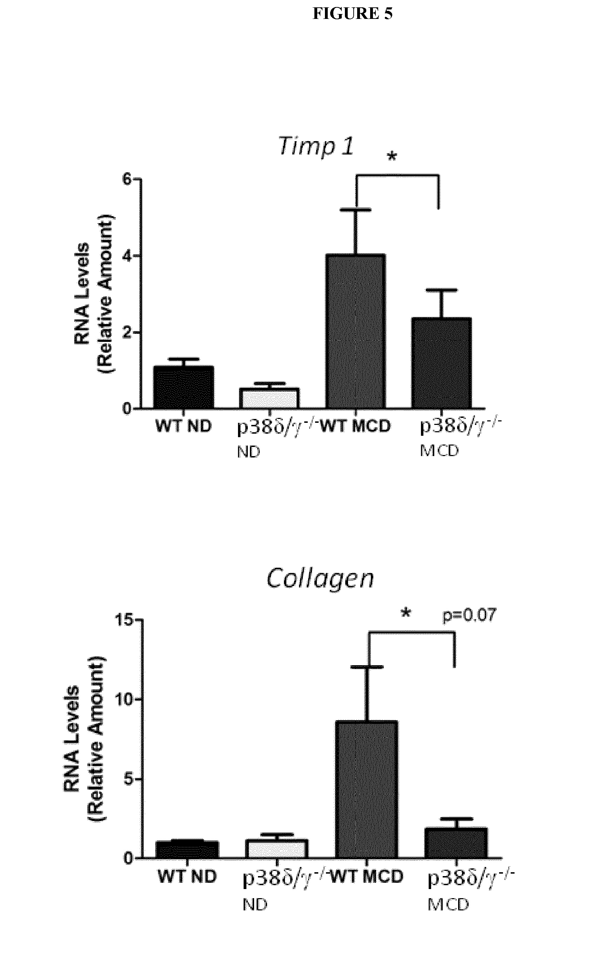

The lower levels of lipid accumulation in liver could be responsible for the reduction in liver inflammation observed in these animals after MCD diet with a decrease in the expression levels of TNF and IL6 (see **Figure 4****,***p < 0.05,**p < 0.01, and ***p < 0.001;by t-test). To study whether all these effects correlate or not with a whole improvement of the disease, levels of liver fibrosis were studied by measuring the levels of TIMP-1 and collagen, two known markers of fibrosis (see **Figure 5****,** *p < 0.05,**p < 0.01, and ***p < 0.001;by *t*-test).). Both markers are increased after MCD diet in the livers of WT animals, but not in the liver from p38δ/γ^{-/-} mice. This indicates that mice defective in p38γ and δ present a great protection against liver steatosis and inflammation.

### Example 3. Effect of the inhibition of p38 MAPK by BIRB796.

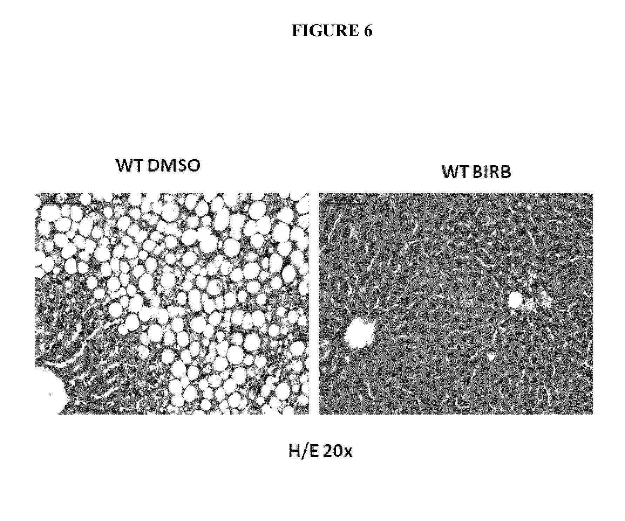

Mice p38γ/δ^{-/-} were obtained as explained in Example 2. Mice fed with MCD diet and treated with BIRB 796 show lower liver steatosis than the ones treated with MCD and vehicle (DMSO). Then it can be concluded that p38γ and δ isoforms are involved in the liver protection of lipid steatosis (see **Figure 6****).**

### Example 4. Mice lacking p38γ and p38δ are resistant to develop liver steatosis during obesity.

Mice p38γ/δ^{-/-} were obtained as explained in Example 2.

Liver extracts were prepared using Triton lysis buffer (20 mM Tris at pH 7.4, 1% Triton X-100, 10% glycerol, 137 mM NaCl, 2 mM EDTA, 25 mM β-glycerophosphate, 1 mM sodium orthovanadate, 1 mM phenylmethylsulfonyl fluoride, 10 µg/mL of aprotinin and 10 µg/mL of leupeptin), and 50 µg of protein examined by immunoblot analysis probing with antibodies against to AKT, phospho-Thr³⁰⁸ AKT, and phospho-Ser⁴⁷³ AKT (Cell Signaling); GAPDH (Santa Cruz Biotechnologies) and SAPK3 (produced by our laboratory).

Tissue fixed in 10% formalin for 24 h, dehydrated, and embedded in paraffin was cut in sections (8 µm) and stained using hematoxylin and eosin or Masson trichromic.

Different histological parameters were analyzed on each liver sample.
- Steatosis: Degree, type and localization.
   ● Degree: Presence of lipid accumulation in vesicles, Mild (<10%), Moderate (10-25%), Severe (26-50%) and Massive (>50%).
   ● Type: Microvesicular, Macrovesicular, and Mixed.
   ● Localization: Periportal, Centrilobular, Diffuse.
- Inflammation: Type and degree.
- Fibrosis: Absent, Perisinusoidal.
- Typical hepatocellular lesions: Presence or absence.

The expression of mRNA was examined by quantitative PCR analysis using a 7900 Fast Real-Time PCR machine. Primers were produced by Sigma Aldrich **(Table 2).** The relative mRNA expression was normalized by measurement of the amount of *Gapdh* mRNA.

**Table 2**

| | Forward | Reverse |
|---|---|---|
| *mmu-p38 delta* | SEQ ID NO: 13 | SEQ ID NO: 14 |
| *mmu-p38 gamma* | SEQ ID NO: 15 | SEQ ID NO: 16 |
| *mmu-Gapdh* | SEQ ID NO: 17 | SEQ ID NO: 18 |

Statistical significance to compare treatment groups was calculated by Student t-test using Prism software (GraphPad). Data were presented as mean±SEM.

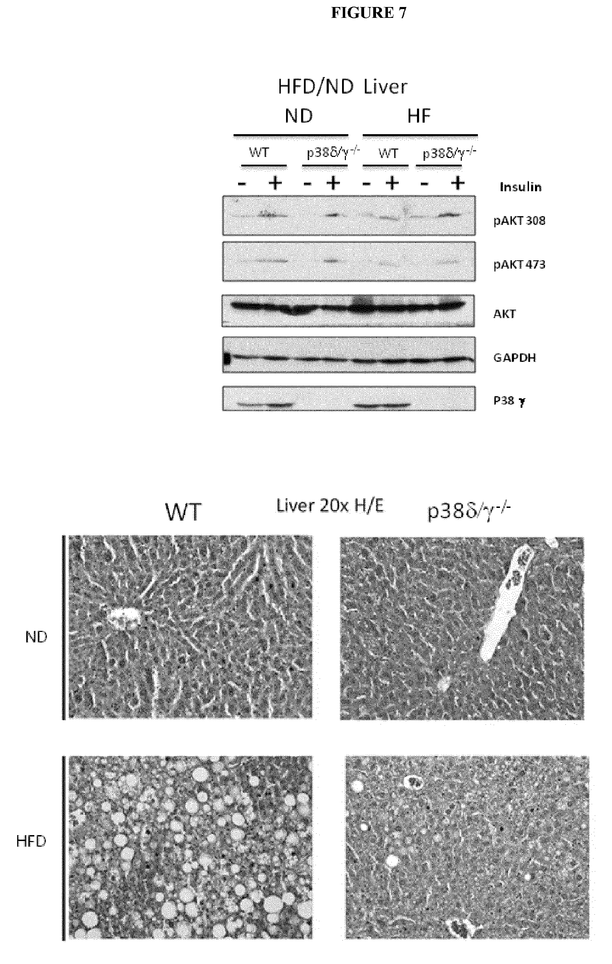

There is a high prevalence of steatosis in obese individuals, it was decided to study the role of p38γ/δ in the development of steatosis in a model of obesity induced esteatosis. Mice were fed with high fat diet (HFD) (60% of the calories from fat) for 20 weeks. After this time, WT mice develop liver steatosis with an increase of lipid accumulation. However, mice lacking p38γ/δ did not accumulate lipid in the liver **(****Figure 7****).** Because liver steatosis induces insulin resistance we decide to study whether the lower lipid accumulation observed in p38γ/*δ*^{-/-} mice correlate with a better insulin response after high fat diet. To confirm the effect steatosis on insulin sensitivity, insulin-stimulated Ser/Thr phosphorylation and activation of AKT were tested. HFD-fed WT mice exhibited reduced insulin-stimulated AKT activation in liver **(Figure 7A).** In contrast, p38γ/δ^{-/-} mice exhibited HFD-induced inhibition of insulin-stimulated AKT activation in muscle, but not in liver (see **Figure 7****).**

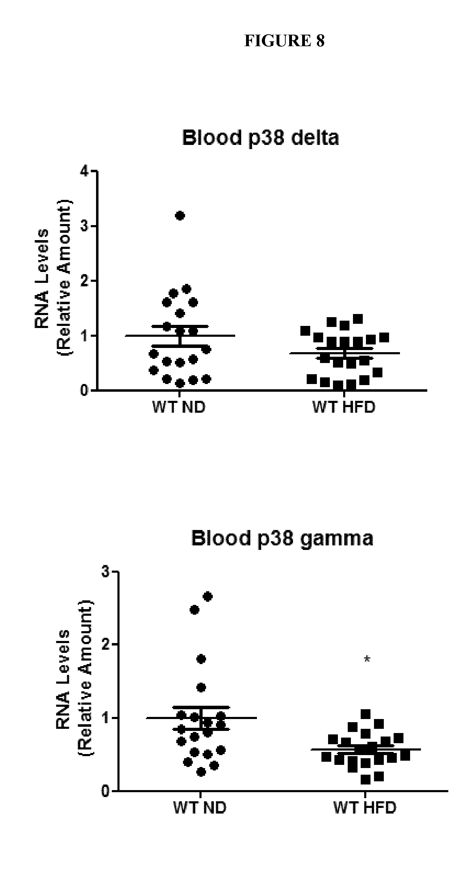

Since the present invention indicates that p38γ/δ have a role on liver steatosis induction, it was decided to measure blood levels of these kinases. Expression levels of these kinases decreased after feeding by HFD **(****Figure 8****),** indicating that they can be used as biomarkers of liver steatosis. *p < 0.05, **p < 0.01, and ***p < 0.001;by *t*-test.

### Example 5. Pathological changes in human obesity.

The study population was comprised of obese adult patients (at least 18 years of age) with body mass index (BMI) greater than 35 who underwent elective bariatric surgery at the University Hospital of Salamanca between November 2010 and November 2011. Patients were excluded if they had prior liver disease, history of alcohol use disorders or excessive alcohol consumption (>30 g/day in men and > 20 g/day in women). Subjects were also excluded if they had chronic hepatitis C or B or if laboratory and histopathological data showed other causes of liver disease different from non-alcoholic fatty liver disease (NAFLD). Patients with body mass index lower than 25 and without liver disease who underwent laparoscopic cholecystectomy were included as a control group. The study was approved by the Ethics Committee of the University Hospital of Salamanca and all subjects provided written informed consent to undergo liver biopsy under direct vision during surgery. A portion of each liver biopsy was fixed in 10% formalin and stained with hematoxylin-eosin and Masson trichrome for standard histopathological interpretation. The remaining portion was immersed in RNAlater (Ambion Inc, USA) and stored at -80°C until RNA was extracted. A sample of subcutaneous and omental adipose tissue were collected as well during surgery. Before surgery, fasting venous blood samples were collected. Serum was obtained after centrifugation and was stored at -80°C and human whole blood was collected in PAXgene Blood RNA Tubes (ref 762165) and stored at -80°C until RNA was extracted.

Data were collected on demographic information (age, sex, and ethnicity), anthropomorphic measurements (BMI), coexisting medical conditions, medication use, smoking and alcohol history, and laboratory data including complete cell blood count, total bilirubin, aspartate aminotransferase (AST), alanine aminotransferase (ALT), alkaline phosphatase, gamma-glutamyl-transpeptidase, total cholesterol, high-density lipoprotein, low-density lipoprotein, triglycerides, creatinine, glucose, glycated haemoglobin, albumin, and iron metabolism markers. Abdominal ultrasound imaging was also obtained before surgery.

Tissue fixed in 10% formalin for 24 h, dehydrated, and embedded in paraffin was cut in sections (8 µm) and stained using hematoxylin and eosin or Masson trichromic.

Different histological parameters were analyzed on each liver sample.
- Steatosis: Degree, type and localization.
   ● Degree: Presence of lipid accumulation in vesicles, Mild (<10%), Moderate (10-25%), Severe (26-50%) and Massive (>50%).
   ● Type: Microvesicular, Macrovesicular, and Mixed.
   ● Localization: Periportal, Centrilobular, Diffuse.
- Inflammation: Type and degree.
- Fibrosis: Absent, Perisinusoidal.
- Typical hepatocellular lesions: Presence or absence.

Statistical significance to compare treatment groups was calculated by Student t-test using Prism software (GraphPad). Data were presented as mean±SEM.

It has been extensively described how a high intake of calories can induce liver damage. The first and most visible change is observed at histological level in obese patients which have a large amount of lipids droplets inside the hepatocytes that can impair the normal function of the liver.

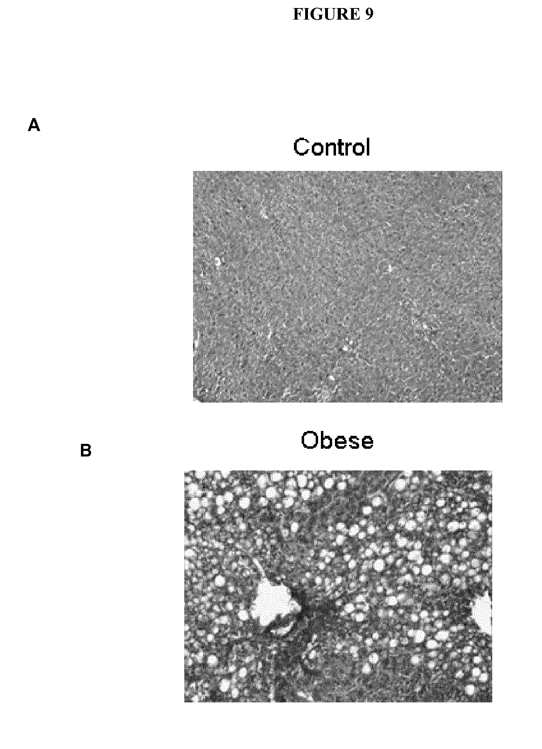

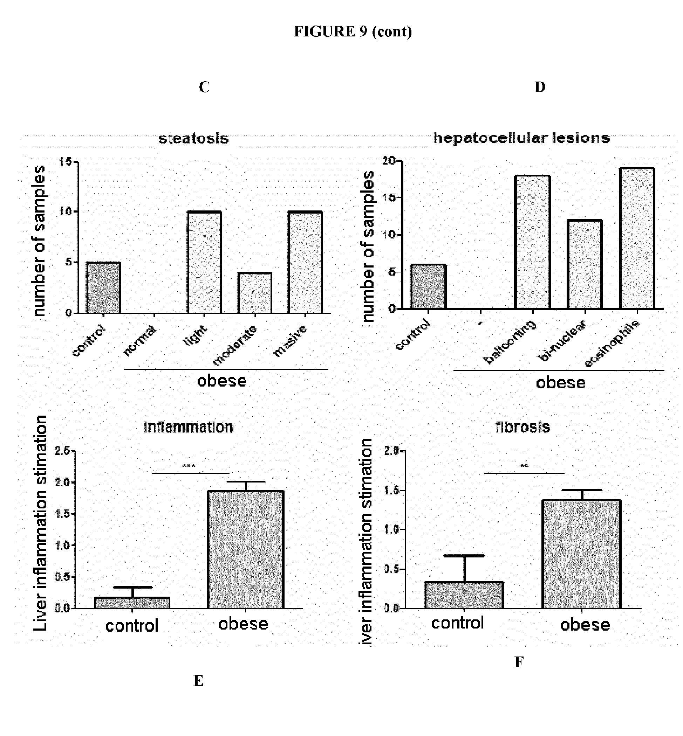

Hepatic fibrosis was assessed by Masson stain. No histological signs of hepatic fibrosis were observed in controls. However, obvious hepatic fibrosis was detected in 92% of obese patients, with different grades of fibrosis. The liver leukocyte infiltration was also detected in all the obese patients ranging from a medium to high grade of mononuclear cell infiltration (MNCs), a change not detected in control patients (see **Figure 9****,** *p < 0.05,**p < 0.01, and ***p < 0.001;by *t*-test ).

### Example 6. p 38γ and δ are potential markers for liver steatosis.

The study population was comprised of obese adult patients (at least 18 years of age) with body mass index (BMI) greater than 35 who underwent elective bariatric surgery at the University Hospital of Salamanca between November 2010 and November 2011. Patients were excluded if they had prior liver disease, history of alcohol use disorders or excessive alcohol consumption (>30 g/day in men and > 20 g/day in women). Subjects were also excluded if they had chronic hepatitis C or B or if laboratory and histopathological data showed other causes of liver disease different from non-alcoholic fatty liver disease (NAFLD). Patients with body mass index lower than 25 and without liver disease who underwent laparoscopic cholecystectomy were included as a control group. The study was approved by the Ethics Committee of the University Hospital of Salamanca and all subjects provided written informed consent to undergo liver biopsy under direct vision during surgery. A portion of each liver biopsy was fixed in 10% formalin and stained with hematoxylin-eosin and Masson trichrome for standard histopathological interpretation. The remaining portion was immersed in RNAlater (Ambion Inc, USA) and stored at -80°C until RNA was extracted. A sample of subcutaneous and omental adipose tissue were collected as well during surgery. Before surgery, fasting venous blood samples were collected. Serum was obtained after centrifugation and was stored at -80°C and human whole blood was collected in PAXgene Blood RNA Tubes (ref 762165) and stored at -80°C until RNA was extracted.

Data were collected on demographic information (age, sex, and ethnicity), anthropomorphic measurements (BMI), coexisting medical conditions, medication use, smoking and alcohol history, and laboratory data including complete cell blood count, total bilirubin, aspartate aminotransferase (AST), alanine aminotransferase (ALT), alkaline phosphatase, gamma-glutamyl-transpeptidase, total cholesterol, high-density lipoprotein, low-density lipoprotein, triglycerides, creatinine, glucose, glycated haemoglobin, albumin, and iron metabolism markers. Abdominal ultrasound imaging was also obtained before surgery.

The expression of mRNA was examined by quantitative PCR analysis using a 7900 Fast Real-Time PCR machine. Primers were designed as shown in **Table 3.** The relative mRNA expression was normalized by measurement of the amount of s18 mRNA.

**Table 3**

| | Forward | Reverse |
|---|---|---|
| h*-p38 beta* | SEQ ID NO: 19 | SEQ ID NO: 20 |
| **h*-p38 gamma*** | **SEQ ID NO: 21** | **SEQ ID NO: 22** |
| **h*-p38 delta*** | **SEQ ID NO: 23** | **SEQ ID NO: 24** |
| h*-s18* | SEQ ID NO: 25 | SEQ ID NO: 26 |

Statistical significance to compare treatment groups was calculated by Student t-test using Prism software (GraphPad). Data were presented as mean±SEM.

Blood samples were collected from obese and lean or healthy patients and the expression levels of p38 gamma and delta were analyzed by Q-PCR. A reduced expression of p38γ and δ of blood levels were found among obese patients. This indicates that p38γ and p38δ are good markers for predicting liver steatosis without an invasive test.

According to the present invention, measuring blood mRNA levels of p38γ and δ in relative amount against s18 for the diagnosis of NAFLD could be of potential clinical use. p38γ levels lower than 0,30 (threshold value) had a sensitivity rate of 83,33% and a specificity rate of 73% for the diagnosis of NALFD, with a positive predictive value (PPV) of 76,92% and a negative predictive value (NPV) of 80,0%. Besides, p38 δ levels lower than 0,45 (threshold value) had a sensitivity rate of 83,33% and a specificity rate of 82,0%, with a PPV of 83,33% and a NPV of 81,82%. The combination of both markers improved specificity (90,9%) but slightly decreased sensitivity (75%).

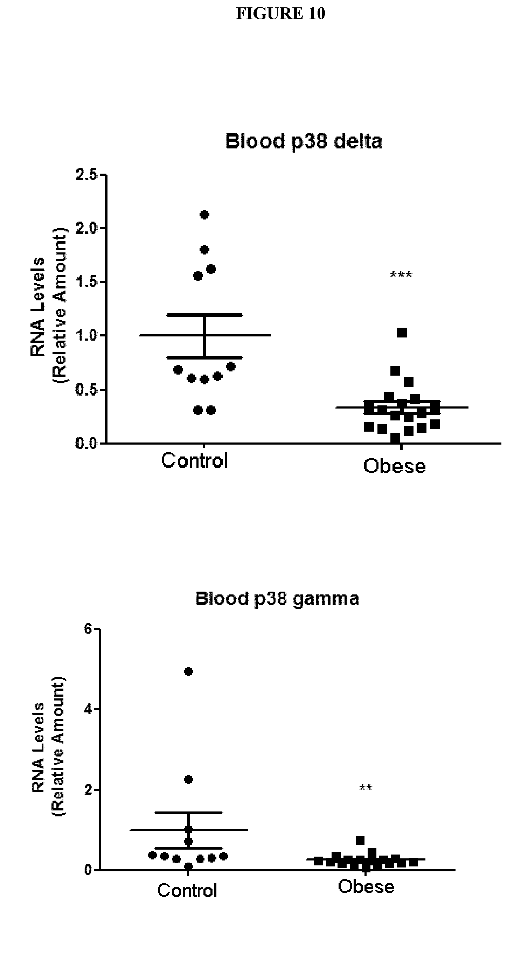

Therefore, the detection of p38γ and δ blood levels could detect the presence of liver steatosis and the combination of these markers with other markers of liver inflammation or liver fibrosis could potentially improve the detection of early fibrosis or monitor the development of cirrhosis (see **Figure 10** *p < 0.05, **p < 0.01, and ***p < < 0.001;by *t*-test).

### References

1. Luyckx FH, Desaive C, Thiry A, Dewé W, Scheen AJ, Gielen JE, Lefèbvre PJ. Liver abnormalities in severely obese subjects: effect of drastic weight loss after gastroplasty, Int J Obes Relat Metab Disord. 1998 Mar;22(3):222-6.
2. Angulo P, Lindor KD. Treatment of non-alcoholic steatohepatitis, Best Pract Res Clin Gastroenterol. 2002 Oct;16(5):797-810.
3. Yatsuji S, Hashimoto E, Tobari M, Taniai M, Tokushige K, Shiratori K., Clinical features and outcomes of cirrhosis due to non-alcoholic steatohepatitis compared with cirrhosis caused by chronic hepatitis C. J Gastroenterol Hepatol. 2009 Feb;24(2):248-54.
4. Sheth, S. G., Gordon, F. D. & Chopra, S. Nonalcoholic steatohepatitis. Ann. Intern. Med. 126,137-145 (1997).
5. Ludwig, J., Viggiano, T. R., McGill, D. B. & Oh, B. J. Nonalcoholic steatohepatitis: Mayo Clinic experiences with a hitherto unnamed disease. Mayo Clin. Proc. 55, 434-438 (1980).
6. Caldwell, S. H. et al. Cryptogenic cirrhosis: clinical characterization and risk factors for underlying disease. Hepatology 29, 664-669 (1999).
7. Shimada, M. et al. Hepatocellular carcinoma in patients with non-alcoholic steatohepatitis. J. Hepatol.37, 154-160 (2002).
8. Propst, A., Propst, T., Judmaier, G. & Vogel, W. Prognosis in nonalcoholic steatohepatitis. Gastroenterology 108, 1607 (1995).
9. Michael M. Van Ness,Anna Mae Diehl, Ann, Is Liver Biopsy Useful in the Evaluation of Patients with Chronically Elevated Liver Enzymes? Intern Med September 15, 1989 111:473-478.
10. Skelly MM, James PD, Ryder SD,Findings on liver biopsy to investigate abnormal liver function tests in the absence of diagnostic serology. J Hepatol. 2001; 35:195-199.
11. Enslen H, Brancho DM, Davis RJ.Molecular determinants that mediate selective activation of p38 MAP kinase isoforms. EMBO J.2000 Mar 15;19(6):1301-11.
12. Remy G, Risco AM, Iñesta-Vaquera FA, González-Terán B, Sabio G, Davis RJ, Cuenda A. Differential activation of p38MAPK isoforms by MKK6 and MKK3, Cell Signal. 2010 Apr;22(4):660-7. Epub 2009 Dec 11.
13. Varela-Rey, M. et al. Non-alcoholic steatohepatitis and animal models: understanding the human disease. Int. J. Biochem. Cell Biol. 41, 969-976 (2009).

## Claims

1. p38MAPK gamma and/or delta for use as biomarkers of NAFLD.

2. p38MAPK gamma and/or delta, according to claim 1, for use as biomarkers of liver steatosis.

3. *In vitro* method for the diagnosis of NAFLD that comprises measuring the expression level of p38MAPK gamma and/or delta in a blood sample taken from the subject under examination.

4. *In vitro* method, according to claim 3, for the diagnosis of liver steatosis.

5. In vitro method, according to claim 3, **characterized in that** the expression level of p38MAPK gamma is measured using the primers of SEQ ID NO: 21 and 22 and the expression level of p38MAPK delta is measured using the primers of SEQ ID NO: 23 and 24.

6. *In vitro* method, according to claim 5, wherein the expression level of p38MAPK gamma and/or delta is reduced in patients suffering from NAFLD as compared with the lean individuals.

7. Nucleotide sequence **characterized by** the SEQ ID NO: 21, 22, 23 or 24.

8. Nucleotide sequence **characterized by** the SEQ ID NO: 21, 22, 23 or 24 or any combination thereof for use as primers for hybridizing under stringent conditions with a nucleic acid encoding for p38MAPK gamma and/or delta sequence or any partial, complementary or mutated sequence thereof.

9. kit for the diagnosis of NAFLD **characterized by** comprising the primers of SEQ ID NO: 21 and 22 for measuring the expression level of p38MAPK gamma and/or the primers of SEQ ID NO: 23 and 24 for measuring the expression level of p38MAPK delta.

10. Kit, according to claim 9, for the diagnosis of liver steatosis.
